# EUROPEAN PATENT APPLICATION

(11) **EP 4 660 323 A1**
(43) Date of publication of application: **10.12.2025**
(21) Application number: 24750214.9
(22) Date of filing: 29.01.2024
(51) Int. Cl.: C12Q 1/6886, C12N 15/12, C12Q 1/686, C12Q 1/6813

(54) **PROGNOSIS PREDICTION METHOD FOR METASTATIC HORMONE-SENSITIVE PROSTATE CANCER**

(30) Priority: 30.01.2023 JP 2023011986
(71) Applicant: Jichi Medical University, Tokyo 102-0093 (JP)
(72) Inventor: FUJIMURA, Tetsuya, Shimotsuke-shi, Tochigi 329-0498 (JP); SUGIHARA, Toru, Shimotsuke-shi, Tochigi 329-0498 (JP); KAMEDA, Tomohiro, Shimotsuke-shi, Tochigi 329-0498 (JP)
(74) Representative: Plougmann Vingtoft a/s
(86) International application number: PCT/JP2024/002666
(87) International publication number: WO 2024/162265

(57) **Abstract**

An object of the present invention is to provide an examination method that enables the prediction of prognosis of metastatic hormone-sensitive prostate cancer highly likely having unsatisfactory prognosis by a means applicable to clinical sites. The present invention relates to an examination method for predicting prognosis of metastatic hormone-sensitive prostate cancer, comprising a step of measuring an expression level of at least one gene selected from an androgen receptor gene and an osteoglycin gene in a prostate tissue taken from a patient with metastatic hormone-sensitive prostate cancer or a transcript thereof.

## Description

### Technical Field

The present invention relates to a method for predicting the prognosis of metastatic hormone-sensitive prostate cancer.

### Background Art

Prostate cancer is the 2nd most common tumor in men and the fifth leading cause of cancer-related death. In most men, the disease is diagnosed as treatable; however, recent epidemiological data indicate that the incidence of metastatic prostate cancer has been increased. Metastatic hormone-sensitive prostate cancer (mHSPC) exhibits a wide variety of resistance to treatment and prediction is difficult.

Clinical indicators such as Gleason score, tumor stage and PSA concentration are used in various combinations in a model for predicting disease outcomes. However, clinical outcomes of individual patients cannot be accurately predicted based on these prognostic indicators. To determine which patients would benefit from more aggressive treatment and which patients can avoid unnecessary and harmful therapeutic interventions, improved biomarkers are required for predicting prognosis.

Some groups have tried to develop tumor-derived RNA expression markers in order to enhance the predictive performance of clinical indicators. However, heretofore, any model for predicting a cancer specific survival (CSS) rate with respect to mHSPC have not been established. The present inventors have successfully extracted high-quality RNA by laser microdissection (technique of cutting a cancer tissue by a laser scalpel under a microscope to extract RNA) of paraffin-fixed specimens obtained by prostate needle biopsy. It has been verified that 10 types of androgen/estrogen signals and a stem-cell marker gene relate to the life prognosis by using two independent cohorts (Non Patent Literature 1). However, the laser microdissection is complicated and takes a long time. Thus, it is difficult to emplo the laser microdissection at clinical sites.

Further,, a method for determining the likelihood of cancer recurrence based on the expression levels of a plurality of microRNAs measured in prostate cancer patients, has been reported (Patent Literature 1).

### Citation List

### Patent Literature

Patent Literature 1: JP-A 2018-68299

### Non Patent Literature

Non Patent Literature 1: Clin Cancer Res. 2014; 20 (17): 4625-35.

### Summary of Invention

### Technical Problem

In the above described Non Patent Literature 1, the life prognosis in the case of mHSPC can be distinguished into three stages (good, intermediate, and poor) but the prediction of accurate survival rates cannot be attained. Further, the laser microdissection employed in the method disclosed in Non Patent Literature 1 is a complicated technique as mentioned above. Thus, it is difficult to employ the laser microdissection at usual clinical sites.

Thus, it is an object of the present invention to provide an examination method that the prognosis of metastatic hormone-sensitive prostate cancer highly likely having unsatisfactory prognosis can be predicted by a means to be employed at clinical sites.

### Solution to Problem

The present inventors have investigated with a view to finding a measurement biomarker for a means to be employed at clinical sites. As a result, they have found that when the expression level of at least one gene selected from an androgen receptor gene and an osteoglycin gene in the prostate tissue taken from a patient with metastatic hormone-sensitive prostate cancer is low, the prognosis of the patient can be potentially fatal. Thus, the present invention has been accomplished.

More specifically, the present invention provides the following [1] to [12].
[1] An examination method for predicting prognosis of metastatic hormone-sensitive prostate cancer, comprising a step of measuring an expression level of at least one gene selected from an androgen receptor gene and an osteoglycin gene, or a transcript thereof, in a prostate tissue taken from a patient with metastatic hormone-sensitive prostate cancer.
[2] The examination method according to [1], comprising a step of measuring the expression levels of the two genes: an androgen receptor gene and an osteoglycin gene, or transcripts thereof.
[3] The examination method according to [1] or [2], further comprising a step of providing information for predicting prognosis of metastatic hormone-sensitive prostate cancer by comparing a measured value of the expression level with a reference value of the gene or a transcript thereof.
[4] The examination method according to any one of [1] to [3], wherein the expression level of the gene or a transcript thereof is an expression level of mRNA.
[5] The examination method according to any one of [1] to [4], wherein the expression level of the gene or a transcript thereof is measured by PCR, northern blot hybridization or a microarray.
[6] The examination method according to any one of [1] to [5], wherein the expression level of the gene or a transcript thereof is measured by RT-PCR.
[7] A companion diagnostic method based on the examination according to any one of [1] to [6].
[8] A reagent for predicting prognosis of metastatic hormone-sensitive prostate cancer comprising an oligonucleotide that specifically hybridizes with at least one gene selected from an androgen receptor gene and an osteoglycin gene or a transcript thereof, or a fragment thereof.
[9] The reagent according to [8], comprising a primer set for amplifying at least one gene selected from an androgen receptor gene and an osteoglycin gene, a transcript thereof or a fragment thereof, or a probe that specifically hybridizes with at least one gene selected from an androgen receptor gene and an osteoglycin gene, or a transcript thereof, or a fragment thereof.
[10] The reagent according to [8] or [9], comprising a primer set for amplifying at least one gene selected from an androgen receptor gene and an osteoglycin gene, or a transcript thereof, or a fragment thereof.
[11] The reagent according to any one of [8] to [10], wherein the two genes: an androgen receptor gene and an osteoglycin gene, or transcripts thereof, or fragments thereof are used.
[12] A companion diagnostic drug comprising the reagent for predicting prognosis according to any one of [8] to [11] .

### Advantageous Effects of Invention

According to the examination method of the present invention, it is possible to predict the prognosis of a metastatic hormone-sensitive prostate cancer, particularly the possibility of death, and the examination method is useful for the preparation of guidelines for therapy.

### Brief Description of Drawings

[Fig.1] Fig.1 illustrates a nomogram for use in predicting a 1-, 3- and 5-year mHSPC-specific survival rate.
[Fig.2] Fig.2 illustrates a Kaplan-Meier curve indicating the relationship between AR (A) and OGN (B) expression levels and prostate cancer specific survival rate.

### Description of Embodiments

In the present invention, the term of "nucleic acid" or "polynucleotide" refers to DNA, RNA or a fragment thereof. Examples of DNA include cDNA, genomic DNA, and synthetic DNA. Examples of "RNA" include total RNA, mRNA, rRNA, tRNA, non-coding RNA and synthetic RNA.

In the present invention, the "gene" includes a double-stranded DNA including human genomic DNA, a single-stranded DNA (positive strand) including cDNA, a single-stranded DNA (complementary strand) having a sequence complementary to the positive strand, and fragments of these, and refers to a nucleotide sequence constituting DNA and containing biological information.

The "gene" in the present invention includes not only a "gene" represented by a specific nucleotide sequence but also a homolog thereof (more specifically, homolog or ortholog), a mutant such as a genetic polymorphism and a derivative thereof.

The names of genes disclosed in the specification follow the Official Symbols specified in the NCBI ([www.ncbi.nlm.nih.gov/]).

In the present invention, the "transcript" of a gene refers to RNA transcribed from a gene (DNA).

An embodiment of the present invention is an examination method for predicting the prognosis of metastatic hormone-sensitive prostate cancer, comprising a step of measuring an expression level of at least one gene selected from an androgen receptor gene and an osteoglycin gene, or a transcript thereof, in a prostate tissue taken from a patient with metastatic hormone-sensitive prostate cancer.

The subject to be examined in the present invention is a patient with metastatic hormone-sensitive prostate cancer (mHSPC).

The "metastatic nature" used herein means diagnosing a person as having distant metastasis such as bone metastasis, lung metastasis and liver metastasis, or distant metastasis in lymph nodes (e.g., neck, mediastinum, abdomen) except the pelvis, which was confirmed by computerized tomography or bone scintigraphy.

The hormone sensitivity refers to a state before an androgen deprivation therapy (ADT) is internally or surgically received, more specifically, a patient administered with an anti-androgen agent or a patient having surgical castration.

The patient with prostate cancer is a patient having adenocarcinoma pathologically confirmed by prostate biopsy.

The biological sample to be used in the present invention is a prostate tissue taken from a patient with metastatic hormone-sensitive prostate cancer. The prostate tissue can be taken by a tissue collection needle commonly employed. The tissue thus taken may be cryopreserved or fixed with formalin for histopathological examination.

A target to be measured by the examination method of the present invention is the expression level of at least one gene selected from an androgen receptor gene and an osteoglycin gene, or a transcript thereof.

That is, the target to be measured is the expression level of at least one gene (DNA) selected from an androgen receptor gene and an osteoglycin gene, or a transcript (RNA) thereof. Further, the expression level refers to the level of expression of the gene or a transcript thereof.

A preferable measurement target is the expression level of RNA of at least one gene selected from an androgen receptor gene and an osteoglycin gene, more preferably the expression level of mRNA of at least one gene selected from an androgen receptor gene and an osteoglycin gene.

Androgen plays an important role in the development and progression of prostate cancer. Androgen binds to an androgen receptor (AR), which is a type of nuclear receptor present in a prostate cell, and migrates into the interior of the cell. Thereafter, androgen binds to an androgen-receptor binding sequence present in a promoter and enhancer region of the target gene, in cooperation with an AR transcription cofactor, to regulate gene expression. AR is expressed in almost all prostate cancer cells. Further, it has been reported that 90% of prostate cancer has androgen-dependent proliferation potency at the initiation of treatment. Thus, the endocrine therapy plays an important role in prostate cancer.

According to the results of the present invention, the decrease in AR expression in a prostate needle biopsy specimen suggests that the cancer-specific survival rate (CSS) of mHSPC patients may decrease. It is important to confirm AR expression before the initiation of therapy.

Osteoglycin (OGN) is a type of proteoglycan. *In vitro* tests have suggested that OGN suppresses bone formation. Further, OGN is expressed not only in muscle and bone but also in a wide variety of organs, and its contribution to various types of malignant diseases has been demonstrated. There are some reports on the relationship between OGN and cancer but no reports on the relationship between OGN and mHSPC.

According to the results of the present invention, it is suggested that if OGN expression is low in a prostate needle biopsy specimen of an mHSPC patient, CSS may decrease.

Examples of a means for measuring the gene or a transcript thereof include PCR, northern blot hybridization and a microarray. Among them, PCR is particularly preferable because it can be measured at clinical sites.

A preferred embodiment of the present invention includes measuring the expression levels of transcripts (RNAs) of the two genes: an androgen receptor gene and osteoglycin gene. In this case, the expression levels of mRNAs contained in a prostate cancer tissue are analyzed. More specifically, RNAs are converted into cDNAs by reverse transcription, and then each of cDNAs or an amplified product thereof is measured.

RNAs can be extracted from a prostate tissue by a method commonly used for extraction or purification of RNA from a biological sample. Examples of the method that can be used include phenol/chloroform method, AGPC (acid guanidinium thiocyanate-phenol-chloroform extraction) method, a method using a column, such as TRIzol (registered trademark), RNeasy (registered trademark) and QIAzol (registered trademark), a method using a special magnetic particle coated with silica, a method using a Solid Phase Reversible Immobilization magnetic particle, and extraction by commercially available RNA extraction reagent, such as ISOGEN or Pure Link (registered trademark).

In the reverse transcription, a primer targeting predetermined RNA to be analyzed may be used; however, a random primer is preferably used to more comprehensively store and analyze a nucleic acid.

For the reverse transcription, a reverse transcriptase or a reverse transcription reagent kit commonly used can be employed. Preferably, a reverse transcriptase or reverse transcription reagent kit having high accuracy and efficiency is used. Examples thereof include M-MLV Reverse Transcriptase and a modified one thereof, or commercially available reverse transcriptase or reverse transcription reagent kit, such as PrimeScript (registered trademark), Reverse Transcriptase series (manufactured by Takara Bio Inc.) and SuperScript (registered trademark) and Reverse Transcriptase series (Thermo Scientific). For example, SuperScript (registered trademark) III Reverse Transcriptase, and SuperScript (registered trademark) VILO cDNA Synthesis kit (both, Thermo Scientific) are preferably used.

In the elongation reaction by the reverse transcription, the temperature is preferably set at 42°C ± 1°C, more preferably 42°C ± 0.5°C, and further preferably 42°C ± 0.25°C, and the reaction time is set at preferably 60 minutes or more, and more preferably from 80 to 120 minutes.

As the RT-PCR, either commercially available 1-step RT-PCR or 2-step RT-PCR may be used. Alternatively, a commercially available quantitative RT-PCR may be employed.

When the expression level of a target gene is measured by northern blot hybridization, for example, a probe DNA is first labeled with a radioisotope or a fluorescent substance. Then, the obtained labeled DNA is hybridized with RNA of a biological sample transferred to, e.g., nylon membrane, in accordance with a routine method. Thereafter, the formed double strand of the labeled DNA and RNA is detected by a signal derived from the label substance.

When the expression level of a target gene is measured by use of a DNA microarray, for example, at least one nucleic acid (cDNA or DNA) derived from a target gene of the present invention is immobilized onto a support of an array. Labeled cDNA or cRNA prepared from mRNA is allowed to bind onto the microarray and then the label on the microarray is detected. In this manner, the expression level of mRNA can be measured.

The nucleic acid to be immobilized onto the array is not limited as long as it hybridizes with a nucleic acid of interest specifically (in other words, with substantially only the nucleic acid) in stringent conditions, and may, for example, be a nucleic acid having the entire sequence of the target gene of the present invention or a nucleic acid consisting of a partial sequence thereof. Herein, the "partial sequence" refers to a nucleic acid consisting of at least 15 to 25 nucleotides. The stringent conditions herein can be usually washing conditions of, e.g., "1 × SSC, 0.1% SDS, 37°C". The more stringent hybridization conditions can be, e.g., "0.5 × SSC, 0.1% SDS, 42°C" and further more stringent hybridization conditions can be, e.g., "0.1 × SSC, 0.1% SDS, 65°C". The hybridization conditions are described in, e.g., J. Sambrook et al., Molecular Cloning: A Laboratory Manual, Third Edition, Cold Spring Harbor Laboratory Press (2001).

In this manner, the expression level of at least one gene selected from an androgen receptor gene and an osteoglycin gene, or a transcript thereof, in a prostate tissue taken from a patient with metastatic hormone-sensitive prostate cancer is measured, and the prognosis of the metastatic hormone-sensitive prostate cancer is predicted based on the expression level. The prognosis is preferably predicted by performing a step of providing information for predicting the prognosis of metastatic hormone-sensitive prostate cancer by comparing the measured value of the expression level to a reference value (cutoff value) of the gene or a transcript thereof.

When the expression level of the gene alone is analyzed by, e.g., RT-PCR, it is preferable to use an analysis method in which the expression level of a target gene is converted into a relative expression level (relative quantification) based on the expression level of a housekeeping gene (e.g., GAPDH gene), or use an analysis method based on an absolute copy number (absolute quantification) determined by using a plasmid containing the region of the gene. Alternatively, the copy number may be obtained by a digital PCR method.

In the "prognosis" in the present invention, the future outcomes of prostate cancer of a patient are all included; however, the present invention is particularly excellent because the cancer-specific survival can be predicted.

Accordingly, the "cutoff value" ("reference value") in the present invention is desirably a reference value based on which the cancer-specific survival can be predicted.

Such a reference value can be determined in advance based on, for example, the relationship between the J-CAPRA score of clinical data on metastatic hormone-sensitive prostate cancer which is conventionally used and the expression level of the gene of the present invention or a transcript thereof.

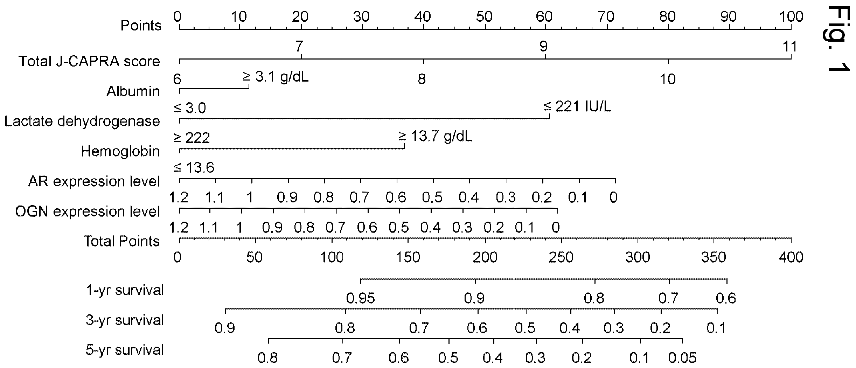

More specifically, the cross-points between points of 0 to 100 in the top stage of Figure 1 and the numerical values of factors such as J-CAPRA score, serum albumin value, a serum LDH value, a blood hemoglobin concentration, an AR expression level, and an osteoglycin expression level of a patient become the points of individual factors. As described above, the present invention is useful as diagnosis of individual patient prognosis, in other words, as companion diagnosis.

More specifically, if the J-CAPRA score is 7, 20 points are given. If the serum albumin value is 3.0, 0 points are given. If the serum LDH value is 221 or less, 60 points are given. If the blood hemoglobin concentration is 13.7 or more, 37.5 points are given. If the AR expression level is 0.7, 30 points are given. If the osteoglycin expression level is 0.3, 47.5 points are given. As a result, the total point of this case becomes 195 points. If the total point is 195, 1-year survival rate, 3-year survival rate, and 5-year survival rate shown in the lower stage of the figure become 90%, 60%, and 45%, respectively. The cancer-specific survival rate in the case of prostate cancer can be estimated from the information at the time of diagnosis.

When the expression level of at least one gene (DNA) selected from an androgen receptor gene and an osteoglycin gene, or a transcript thereof (RNA) is higher than the reference value thereof, the cancer-specific survival can be predicted.

Another embodiment of the present invention is a reagent for predicting the prognosis of the metastatic hormone-sensitive prostate cancer, wherein the reagent comprises an oligonucleotide that specifically hybridizes with at least one gene selected from an androgen receptor gene and an osteoglycin gene, or a transcript thereof, or a fragment thereof.

Examples of the oligonucleotide specifically hybridizing with the gene, a transcript thereof or a fragment thereof used in the reagent of the present invention include a primer set and a probe used in PCR. The primer set is more preferable. The nucleotide sequences of the androgen receptor gene and osteoglycin gene are already known and a primer set for amplifying these genes is already known.

The reagent may contain, other than the above primer set, e.g., a labeling reagent, a buffer, a coloring substrate, an instrument required for a test, control reagents such as a positive control and a negative control, and storage containers.

The present invention is useful for companion diagnosis associated with the prognosis prediction of metastatic hormone-sensitive prostate cancer. The companion diagnosis is a clinical test for predicting the effect and a side effect of a drug before administration, and is used for promoting personalized medicine (or custom-made medicine) by examining the reactivity of a patient to a drug before treatment.

If the present invention is applied to a hypothetical case, the selection of drug therapy can be appropriately made at the time of diagnosis.

More specifically, in a case where a serum PSA value at diagnosis is 500 ng/dL, biopsy tissue Gleason score is 4 + 4 cT3a N1, and bone metastasis is observed, JCAPRA score becomes 2 + 3 + 2 + 1 + 3 = 11. In a case where serum the albumin value is 3.5 g/dL, LDH is 220 IU/L, hemoglobin concentration is 10 g/dL, AR expression level is 0.5, and OGN expression level is 0.6, the total point becomes 100 + 10 + 60 + 0 + 40 + 30 = 240. These are applied to the nomogram of the present invention, the 1-, 3- and 5-year survival rates become about 85%, 45% and 28%, respectively. When these percentages are compared to 5-year survival rate of 66.7% shown in the prognostic curve provided by the multicenter joint study previously reported, they are extremely bad. Because of this, in this hypothetical case, other than an androgen deprivation therapy commonly applied, a new androgen preparation (abiraterone, Xtandi) or 3-drug combination therapy (androgen deprivation therapy, androgen preparation and taxane anticancer drug) can be diagnosed as a treatment candidate as the initial treatment.

### Examples

Now, the present invention will be described in detail with reference to Examples, however, the present invention is by no means limited thereto.

### Example 1

### (Materials and Methods)

In this examination, formalin-fixed paraffin-embedded sections of primary tumors were used, which were obtained from 103 male patients having no treatment history and diagnosed as mHSPC by ultrasound-guided biopsy at the Jichi Medical University Hospital from 2006 to 2018. As a model test, a cohort of the Jichi Medical University Hospital was used. To all cases, the androgen deprivation therapy (ADT) by internal or surgical castration was applied and, if necessary, an anti-androgen agent was administered. After PSA relapsed during the initial ADT, a further treatment was determined by a physician.

Clinical data including age, physical and clinical test data, and pathological records were collected. Gleason score, PSA and clinical TNM stage were converted into scores of the Japan Cancer of the Prostate Risk Assessment (J-CAPRA), which is an efficient risk assessment tool for predicting ADT outcome of a prostate cancer patient. J-CAPRA scores: biopsy Gleason score (3 + 3: 0 points, 3 + 4 and + 4 + 3: 1 point, 8 +: 2 points), PSA value (0 to 20 ng/mL: 0 points, 20 to 100 ng/mL: 1 point, 100 to 500 ng/mL: 2 points, 500 ng/mL or more: 3 points), clinical stage T stage (cT1a to cT2a: 0 points, cT2b to cT3a: 1 point, cT3b: 2 points, cT4: 3 points), clinical N stage (N0: 0 points, N1: 1 point), clinical M stage (M0: 0 points, M1: 3 points).

The total RNA was isolated by use of Pure Link FFPE Total RNA Isolation Kit (Thermo Fisher, Santa Clara, CA, USA). From 10 paraffin sections of 10 µm in thickness, excess paraffin except for that present in a tissue was removed and 300 µl of Melting Buffer was added. Thereafter, the mixture was centrifuged for 10 seconds and incubated at 72°C for 10 minutes. Proteinase K (20 µl) was added, and the mixture was incubated at 60°C until paraffin was completely dissolved. After one-minute centrifugation, a liquid alone was aspirated. Binding Buffer (400 µl) and 800 µl of 100 % ethanol were added and a bolt was attached. The mixture was centrifuged for one minute. The resulting mixture was transferred to a collection tube having a cartridge. The collection tube was centrifuged at a maximum speed for 2 minutes and the liquid was discarded. This operation was repeated twice. A mixture (500 µL) of Wash Buffer and ethanol was added and centrifuged at a maximum speed for one minute. The liquid was discarded. This operation was repeated three times. The cartridge alone was transferred to an unused tube and 50 µl of RNA-free water warmed up to 65°C was added. Incubation was performed at room temperature for one minute. Centrifugation was made at a maximum speed for one minute to extract RNA. The total RNA was treated with DNase I before cDNA synthesis. The quality and quantity of RNA were evaluated by a nanodroplet spectrophotometer (Thermo Fisher, Santa Clara, CA, USA). The total RNA was converted into a single-stranded cDNA by use of PrimeScript RT Master Mix (Takara). Using the StepOne system, Quantitative reverse transcription PCR was performed.

The PCR conditions were: initial 20 seconds at 95°C, one second at 95°C and 20 seconds at 60°C, 40 cycles.

The IDs of the assays used herein are as follows:
Androgen receptor (AR): Hs00171172
Octamer transcription factor 1 (Oct1): Hs00427552
Kruppel-like factor 4 (Klf4): Hs0035836
Sulfatase 1 (SULF1): Hs00392834
Osteoglycin (OGN): Hs00247901
S100 calcium-binding protein A6 (S100A6): Hs00170953
Sex-determining region Y-box 2 (Sox2): Hs01053049
MYC proto-oncogene, bHLH transcription factor (Cmyc): Hs00153408
Tertile motif containing 36 (TRIM36): Hs01120401
Glyceraldehyde-3-phosphate dehydrogenase (GAPDH): Hs03929097
Estrogen receptor alpha (ERα): Hs01046816

The AR gene regulates the growth of the prostate and is expressed in different stages of prostate cancer. In this study, AR-related genes TRIM36 and Oct1 were also selected with reference to previous reports.

Stem cell-like marker Sox2, Klf4, and C-Myc were also evaluated. SULF1, a gene related to a prostate stromal cell, and a prostate basal cell marker S100A6 were also evaluated. A prostate cancer-related gene ERα, principally expressed in the prostate stroma was also evaluated. OGN, which was suggested to inhibit bone formation in an *in-vitro* test, was also evaluated.

### (Ethical approval)

The ethical approval of this examination was obtained from the ethics committee of the Jichi Medical University in accordance with the opt-out policy (No. 21-002). Written informed consent for publishing the details was obtained from patients.

### (Statistics)

The receiver operating characteristic (ROC) analysis was used to determine the best cutoff value for dichotomize the level of gene expression for predicting CSS. CSS was evaluated by the Kaplan-Meier method and compared by the log-rank test. CSS was defined as the interval of time from the diagnosis of prostate cancer to cancer-related death. Using the univariate Cox proportional hazards analysis for CSS, clinicopathological parameters were compared to a gene expression profile. The factor having a p value of < 0.10 in the Univariate analysis was selected as a candidate to be subjected to the multivariate analysis by the Cox hazard model for CSS.

Based on the results of the multivariate analysis, a nomogram was constructed for evaluating 1-, 3- and 5-year probability of CSS in mHSPC patients. The performance of the nomogram constructed herein was evaluated by the concordance index (C-index). External validation of the nomogram newly established was performed by use of validation cohort in which the gap between the nomogram prediction and the outcome observed was evaluated by C-index.

Statistical analysis was performed using the graphical user interface EZR (Saitama Medical University, Jichi Medical University, Japan, Saitama) of R (the R Foundation for Statistical Computing, Vienna, Austria).

### (Results)

The average value of the resultant total RNA was 1916.0 ng (235.0 to 7880.0). The background of mHSPC patients in 2 facilities were shown in (Table 1). The median of the follow-up period was 36.9 months (range 3.3 to 153.1). The anti-androgen agents used in the Jichi Medical University Hospital cohort are bicalutamide in 79 cases (76.7%), chlormadinone acetate in 14 cases (13.6%), and Odyne in 10 cases (9.7%).

The potential predictors of CSS were evaluated in a model test cohort (n = 103) (Table 2). In the univariate analysis using Cox proportional hazards model, Gleason score, hemoglobin (≥ 13.7 g/dL or < 13.7), serum albumin (≥3.1 g/dL or < 3.1), serum lactate dehydrogenase (≥ 222 IU/L or non), the total J-CAPRA score, AR expression level, and OGN expression level related to CSS (individual HR; 0.62, 0.40, 1.78, 1.17, 0.57, and 0.53; all p value ≤ 0.1). Among these 6 factors, the Gleason score was excluded since the score was included in J-CAPRA score. Nomograms for 1-, 3-, 5-year CSS were constructed using the other 5 factors (Figure 1). The C-index of the nomograms was 0.664.

The background factors of mHSPC103 case are shown in Table 1. The factors (the number of cases = 103) related to cancer-specific survival by Cox proportional hazards model are shown in Table 2.

**[Table 1]**

| Table 1 Clinical background factors of mHSPC103 case | | |
|---|---|---|
| Clinical background factors | | Test group (number of cases = 103) number of cases (%), median value (range) |
| Serum PSA value, ng/mL | | 300.3 (5.56 - 10946.0) |
| Age | | 70.0 (49 - 86) |
| Hemoglobin concentration, g/dL | | 13.2 (7.9 - 17.5) |
| | ≥ 13.7 g/dL, n (%) | 41 (39.8) |
| | < 13.7 g/dL, n (%) | 62 (60.2) |
| Serum albumin value, mg/dL | | 4.0 (2.4 - 5.2) |
| | > 3.1 g/dL, n (%) | 97 (94.2) |
| | < 3.1 g/dL, n (%) | 6 (5.8) |
| Serum lactate dehydrogenase, IU/L | | 210.0 (143.0 - 1456.0) |
| | ≥ 222 IU/L, n (%) | 43 (41.7) |
| | < 222 IU/L, n (%) | 60 (58.3) |
| Alkaline phosphatase, IU/L | | 426.0 (156.0 - 10914.0) |
| | ≥ 113 IU/L, n (%) | 103 (100) |
| | < 113, n (%) | 0 (0) |
| J-CAPRA score | | 10 (4 - 12) |
| Gleason score (J-CAPRA point) | | |
| | 6 (0 point) | 2 (1.9) |
| | 7 (1 point) | 10 (9.7) |
| | 8+ (2 point) | 91 (88.3) |
| Clinical stage T stage (J-CAPRA point) | | |
| | T1a - T2a (0 point) | 3 (2.9) |
| | T2b - T3a (1 point) | 41 (39.8) |
| | 3b (2 point) | 29 (28.2) |
| | 4 (3 point) | 30 (29.1) |
| Clinical stage N stage (J-CAPRA point) | | |
| | N0 (0 point) | 42 (40.8) |
| | N1 (1 point) | 61 (59.2) |
| Metastatic lesion (J-CAPRA point) | | |
| | No metastasis (0 points) | 0 (0) |
| | Metastasis to bone (3 points) | 96 (93.2) |
| | Metastasis to lung (3 points) | 17 (16.5) |
| | Metastasis to lymph node (3 points) | 33 (32.0) |
| | Metastasis to liver (3 points) | 3 (2.9) |

**[Table 2]**

| Table 2 Factors related to cancer-specific survival by Cox proportional hazards model (number of cases = 103) | | | | |
|---|---|---|---|---|
| | Univariate analysis | | multivariate analysis | |
| | Hazard day (95% confidence interval) | P value | Hazard day (95% confidence interval) | P value |
| Age † | 0.99 (0.95-1.02) | 0.48 | | |
| Serum PSA value, ng/mL † | 1.00 (1-1) | 0.35 | | |
| Gleason score † | 1.32 (0.98-1.78) | 0.071 | | |
| Hemoglobin concentration ≥ 13.7 g/dL (vs. < 13.7) | 0.62 (0.35 - 1.07) | 0.09 | 0.7 (0.39 - 1.26) | 0.23 |
| Serum albumin value ≥ 3.1 g/dL (vs. < 3.1) | 0.405 (0.17 - 0.95) | 0.04 | 1.12 (0.45 - 2.75) | 0.81 |
| Serum lactate dehydrogenase ≥ 222 IU/L (vs. < 222) | 1.78 (1.04 - 3.05) | 0.03 | 1.79 (1.02-3.14) | 0.04 |
| Alkaline phosphatase ≥ 113 IU/L (vs. < 113) | NA | NA | | |
| J-CAPRA score † | 1.17 (1-1.37) | 0.04 | 1.21 (1.03-1.43) | 0.02 |
| Gene expression level | | | | |
| *AR†* | 0.57 (0.33-0.99) | 0.04 | 0.57 (0.32-1.43) | 0.05 |
| *OCT1 †* | 1.20 (0.44-3.28) | 0.73 | | |
| *Cmyc †* | 1.01 (0.62-1.65) | 0.96 | | |
| *Sox2 †* | 0.83 (0.52-1.31) | 0.42 | | |
| *Klf4 †* | 1.04 (0.55-1.99) | 0.9 | | |
| *SULF1 †* | 1.69 (0.71-4.03) | 0.24 | | |
| *S100A6 †* | 1.06 (0.86-1.31) | 0.59 | | |
| *TRIM36 †* | 0.99 (0.62-1.58) | 0.96 | | |
| *OGN †* | 0.53 (0.32-0.87) | 0.01 | 0.60 (0.36-1.02) | 0.06 |
| *ERα †* | 1.13 (0.66-1.94) | 0.66 | | |

| | | | | |
|---|---|---|---|---|
| t Continuous variables | | | | |

Appropriate cutoff values of individual genes were determined by preparing an ROC curve including OGN and AR. The cutoff values of OGN and AR were 1.133 and 0.00, respectively.

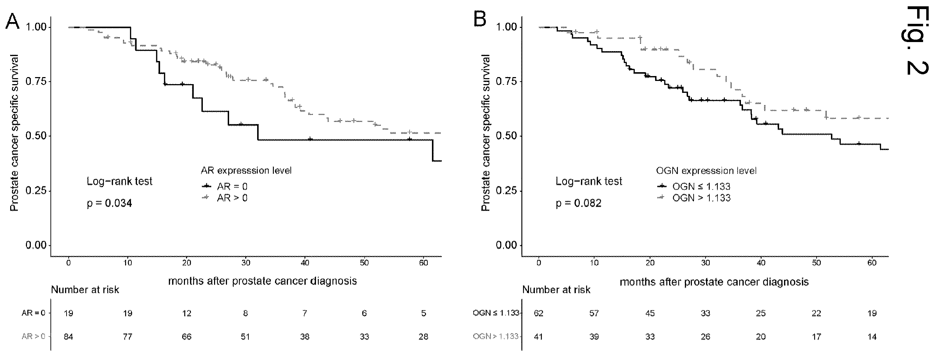

The patients were divided into 2 groups having OGN > 1.133, and OGN ≤ 1.133, and AR > 0.00, AR = 0.00, respectively. The Kaplan-Meier curve of CSS is shown (Figure 2). The longer CSS was observed in the group of OGN > 1.133 (CSS median, 85.3 vs 52.7 months, p = 0.082) and a group of AR > 0.00 (CSS median, 69.1 months vs 32.1 months, p = 0.034).

The analysis of the Human Protein Atlas indicates that the fragments (FPKM) per exon (1 kilobase) per AR and OGN (1 million reads) in prostate cancer are 15.1 (5.1-33.7) and 6.4 (0.0-24.4), respectively.

The androgen receptor and osteoglycin are predictive factors for cancer-specific survival in hormone sensitive prostate cancer. A nomogram for predicting CSS was established by integrating them.

## Claims

1. An examination method for predicting prognosis of metastatic hormone-sensitive prostate cancer, comprising a step of measuring an expression level of at least one gene selected from an androgen receptor gene and an osteoglycin gene, or a transcript thereof, in a prostate tissue taken from a patient with metastatic hormone-sensitive prostate cancer.

2. The examination method according to claim 1, comprising a step of measuring the expression levels of the two genes: an androgen receptor gene and an osteoglycin gene, or transcripts thereof.

3. The examination method according to claim 1 or 2, further comprising a step of providing information for predicting prognosis of metastatic hormone-sensitive prostate cancer by comparing a measured value of the expression level with a reference value of the gene or a transcript thereof.

4. The examination method according to any one of claims 1 to 3, wherein the expression level of the gene or a transcript thereof is an expression level of mRNA.

5. The examination method according to any one of claims 1 to 4, wherein the expression level of the gene or a transcript thereof is measured by PCR, northern blot hybridization or a microarray.

6. The examination method according to any one of claims 1 to 5, wherein the expression level of the gene or a transcript thereof is measured by RT-PCR.

7. A companion diagnostic method based on the examination according to any one of claims 1 to 6.

8. A reagent for predicting prognosis of metastatic hormone-sensitive prostate cancer, comprising an oligonucleotide that specifically hybridizes with at least one gene selected from an androgen receptor gene and an osteoglycin gene, or a transcript thereof, or a fragment thereof.

9. The reagent according to claim 8, comprising a primer set for amplifying at least one gene selected from an androgen receptor gene and an osteoglycin gene, or a transcript thereof or a fragment thereof, or a probe that specifically hybridizes with at least one gene selected from an androgen receptor gene and an osteoglycin gene, or a transcript thereof, or a fragment thereof.

10. The reagent according to claim 8, comprising a primer set for amplifying at least one gene selected from an androgen receptor gene and an osteoglycin gene, or a transcript thereof, or a fragment thereof.

11. The reagent according to any one of claims 8 to 10, wherein the two genes: an androgen receptor gene and an osteoglycin gene, or transcripts thereof, or fragments thereof are used.

12. A companion diagnostic drug comprising the reagent for predicting prognosis according to any one of claims 8 to 11.
